# EUROPEAN PATENT APPLICATION

(11) **EP 1 787 571 A1**
(43) Date of publication of application: **23.05.2007**
(21) Application number: 05767431.9
(22) Date of filing: 28.07.2005
(51) Int. Cl.: A61B 1/00, A61B 1/06, F21V 8/00, H01L 33/00, F21W 131/20, F21Y 101/02

(54) **LIGHT SOURCE AND ENDOSCOPE EQUIPPED WITH THIS LIGHT SOURCE**

(30) Priority: 28.07.2004 JP 2004220725
(71) Applicant: Kyocera Corporation, Kyoto-shi, Kyoto-fu 612-8501 (JP)
(72) Inventor: AJIMA, Hiromi;, Tamagawadai 2-chome, Setagaya-ku, Tokyo (JP); KATABE, Kousuke;, awai, Gamo-cho, Gamo-gun, Shiga 5291595; (JP)
(74) Representative: HOFFMANN EITLE
(86) International application number: PCT/JP2005/013871
(87) International publication number: WO 2006/011571

(57) **Abstract**

A light source apparatus of the present invention comprises a semiconductor light emitting device which emits light, a wavelength converting member which receives the light emitted by the semiconductor light emitting device and emits light of a wavelength longer than that of the original light, and a wavelength selecting member which is disposed between the semiconductor light emitting device and the wavelength converting member and reflects and transmits the incident light with different reflectivity and different transmittance depending on the wavelength of the incident light.

## Description

### FIELD OF THE INVENTION

The present invention relates to a light source apparatus and, more particularly, to a light source apparatus used in industrial and medical applications, and an endoscope provided with the light source apparatus.

### PRIOR ART

An endoscope for medical use comprises an illumination' optical system for illuminating the inside of a body cavity with white light, and a CCD camera for capturing an image of an organ of the body. The illumination optical system is constituted from a high brightness lamp such as xenon lamp, a condenser lens for concentrating the light emitted by the high brightness lamp, a light guide consisting of optical fiber for guiding the light concentrated by the condenser lens and introducing the light into the body cavity, and an illumination lens for illuminating the inside of the body cavity with the light introduced by the light guide. An illumination optical system having such a constitution is disclosed, for example, in "Optical System: Constitution and Applications", Optronics Inc. Editing office, pp205-214 (2003).

In the illumination optical system disclosed in "Optical System: Constitution and Applications", Optronics Inc. Editing office, pp205-214 (2003), a multi-component glass fiber having numerical aperture (NA) of about 0.6 is used as the light guide, and the value of NA is converted to about 0.87 by using an illumination lens provided at the light emerging end of the light guide fiber located at the distal end of the endoscope, for the purpose of illuminating uniformly over the field of view.

However, the illumination optical system of the prior art as described above has such problems that the high brightness lamp generates a large amount of heat, consumes a significant amount of electric power, and has a large overall size.

In order to solve the problems, it has been proposed to use an illumination optical system which employs LED or the like. Light source apparatuses having an illumination optical system which employs LED are disclosed, for example, in Japanese Unexamined Patent Publication (Kokai) No. 2003-235796 and Japanese Unexamined Patent Publication (Kokai) No. 2003-19112. The illumination optical system of the light source apparatus disclosed in patent document 1 has a plurality of LEDs and a reflecting member, so as to introduce the light emitted by the plurality of LEDs into an optical guide member (light guide). The illumination optical system of the light source apparatus disclosed in "Next generation white LED", proceeding of symposium of Applied Physics Association of Japan, Kansai Branch, pp35-40 (November, 2003) has a plurality of solid state light emitting devices (LED, LD, SLD, etc.) and is constituted so as to introduce the light emitted by the plurality of solid state light emitting devices into a fluorescent fiber.

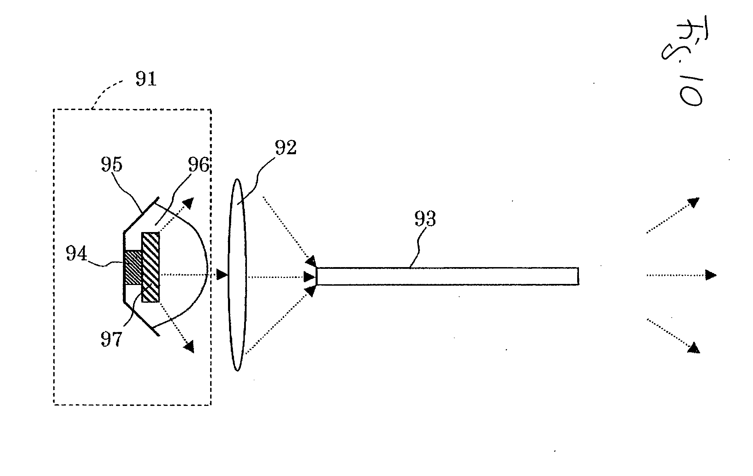

Fig. 10 shows an example of a light source apparatus of the prior art. The light source apparatus comprises a white LED 91, a condenser lens 92 and an optical fiber (POF) 93 made of plastics, so as to concentrate the light emitted by the white LED 91 with the condenser lens 92 and introduce the light into the POF 93 at the input end thereof. The white LED 91 comprises a blue light emitting LED chip 94, a reflector mirror 95, a sealing member 96 and a phosphor 97.

While the white LED 91 used of the light source apparatus of the prior art is smaller in size and consumes less power than the high brightness lamp described above, the output power of light emission and color rendering property are not sufficient in some applications, especially in medical use. Specifically, while non-patent document 2 describes that the white LED constituted from the combination of a blue light emitting LED and a yellow phosphor has a relatively output power of light emission, it may not provide sufficient color rendering property since green component and red component of the light have relatively low intensities by nature.

Accordingly, efforts have been made to develop a white LED having an emission spectrum similar to that of sun light, such as a white LED which combines an ultraviolet ray emitting LED and RGB phosphors, in order to achieve relatively high intensities of green component and red component and high color rendering property.

### DISCLOSURE OF THE INVENTION

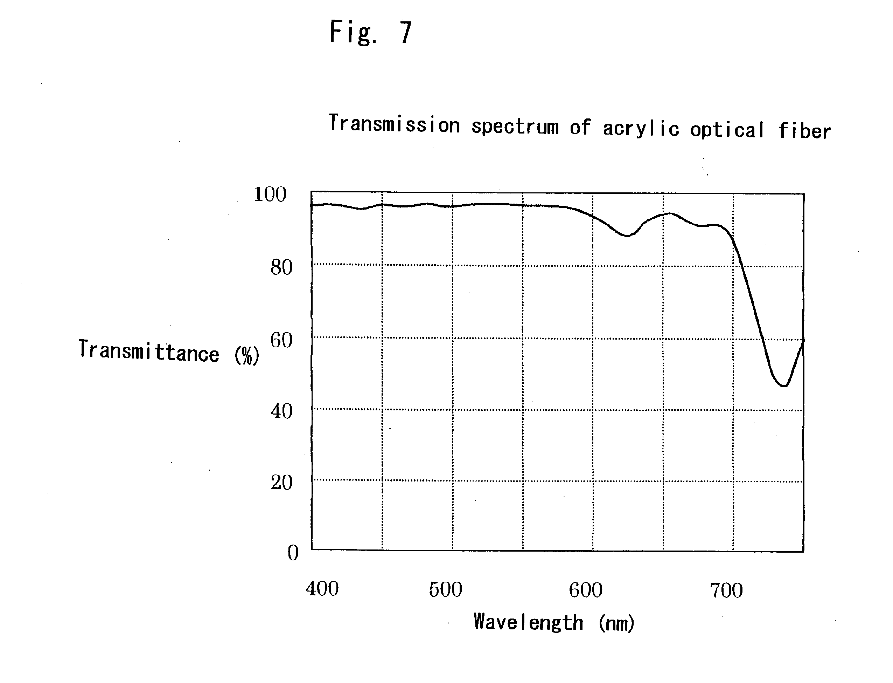

Despite improvements in the color rendering property of the white LED 91 of the light source apparatus of the prior art, light emitted by the white LED 91 emerges through the POF 93 to the outside, and therefore the emission spectrum varies due to the wavelength dependency of transmission loss of the POF 93. Thus satisfactory color rendering property may not be obtained with the light emitted from the light source apparatus of the prior art to the outside, even when the color rendering property of the white LED 91 is improved. Fig. 7 shows the transmission spectrum of an optical fiber made of an acrylic resin. This spectrum indicates that the optical fiber made of an acrylic resin has a relatively large transmission loss of about 10% around red component (wavelength of, for example, 630 nm), while the transmission loss for blue component (wavelength of, for example, 450 nm) has relatively small value of about 2%. Since the transmission loss of the POF 93 has such a wavelength dependency, it is necessary to take into account the constitution of the white LED 91 and the transmission loss of the POF 93 in the design of the light source apparatus of the prior art when it is required to emit light of high color rendering property.

The present invention has been made with the background described above, and has an object of providing a light source apparatus which, in addition to small size and low power consumption, has high output power and high color rendering property, and an endoscope provided with the light source apparatus.

A light source apparatus according to first aspect of the present invention comprises a semiconductor light emitting device which emits light, a wavelength converting member which receives the light emitted by the semiconductor light emitting device and emits light of a wavelength longer than that of the original light, and a wavelength selecting member which is disposed between the semiconductor light emitting device and the wavelength converting member and reflects and transmits the incident light with different reflectivity and different transmittance depending on the wavelength of the incident light.

A light source apparatus according to second aspect of the present invention comprises a semiconductor light emitting device which emits light, a wavelength converting member which receives the light emitted by the semiconductor light emitting device and emits light of wavelength longer than the original light and a light guide member which guides the light emitted by the semiconductor light emitting device to the wavelength converting member.

An endoscope of third aspect of the present invention comprises the light source apparatus of the first or second aspect of the present invention and an image pickup device which captures the image of an area illuminated by the light emitted by the light source apparatus.

The light source apparatus according to the first aspect of the present invention has the wavelength selecting member which is disposed between the semiconductor light emitting device and the wavelength converting member, and reflects and transmits the incident light with different reflectivity and different transmittance depending on the wavelength of the incident light. Therefore, the light source apparatus allows the light emitted by the semiconductor light emitting device to pass through the wavelength selecting member and reach the wavelength converting member efficiently by setting a high value of transmittance of the wavelength selecting member for the light emitted by the semiconductor light emitting device and setting a high value of reflectivity of the wavelength selecting member for light emerging from the wavelength converting member, and enables the wavelength selecting member to effectively reflect the light directed toward the wavelength selecting member among the light emerging from the wavelength converting member. As a result, since the light emerging from the wavelength converting member can be effectively extracted in the direction opposite to the wavelength selecting member in the light source apparatus of the present invention, the efficiency of extracting light in this direction can be improved.

With this light source apparatus, even when a member having transmission loss that is dependent on the wavelength is interposed between the semiconductor light emitting device and the wavelength converting member, the light emerging from the wavelength converting member can be extracted to the outside without passing through the member. Therefore, this light source apparatus is preferable in improving the color rendering property of the light extracted therefrom.

The light source apparatus according to the second aspect of the present invention is constituted so that light emitted by the semiconductor light emitting device is introduced into the wavelength converting member via the light guide member. Accordingly, the light emerging from the wavelength converting member can be extracted to the outside without passing through the light guide member. As a result, this light source apparatus is capable of improving the color rendering property of the light extracted therefrom, even when the light guide member has transmission loss which is dependent on the wavelength.

The endoscope according to the third aspect of the present invention has the light source apparatus of the first or second aspect of the present invention. Therefore, the endoscope is capable of illuminating a body organ with light of high color rendering property. As a result, the endoscope is preferable in perceiving the situation of the organ by the color.

### BRIEF DESCRIPTION OF THE DRAWINGS

Fig. 1 schematically shows the constitution of the light source apparatus according to the first embodiment of the present invention.
Fig. 2A is a diagram showing an angle over which the output light spreads in case the output end of the optical fiber has flat configuration in the light source apparatus shown in Fig. 1.
Fig. 2B is a diagram showing an angle over which the output light spreads in case the output end of the optical fiber has concave configuration in the light source apparatus shown in Fig. 1.
Fig. 3 schematically shows the constitution of the light source apparatus according to the second embodiment of the present invention.
Fig. 4 is a partial sectional view schematically showing the constitution of the endoscope according to the third embodiment of the present invention.
Fig. 5 is a partial sectional view schematically showing the constitution of the endoscope according to the fourth embodiment of the present invention.
Fig. 6 is a diagram showing the emission spectrum of a violet light emitting LED.
Fig. 7 is a diagram showing the spectrum of transmission loss of an optical fiber made of an acrylic resin.
Fig. 8 is a diagram showing the transmission and reflection spectra of wavelength selecting filter.
Fig. 9 is a diagram showing the emission spectrum of a white LED which combines an ultraviolet ray emitting LED and phosphor.
Fig. 10 is a diagram schematically showing the constitution of the light source apparatus of the prior art.

### [DESCRIPTION OF REFERENCE NUMERALS]

- X1, X2:: light source apparatus
- Y1, Y2:: endoscope
- 10:: light source (light emitting diode)
- 11:: semiconductor light emitting device (LED chip)
- 12:: reflecting member (reflector mirror)
- 13:: sealing member
- 20:: condenser lens
- 30:: light guide member (optical fiber)
- 40:: wavelength selecting member (wavelength selecting mirror)
- 50:: wavelength converting member (phosphor-including member)
- 110:: light source connector
- 120:: image pickup device mounting section
- 130:: branching means

### MODE FOR CARRYING OUT THE INVENTION

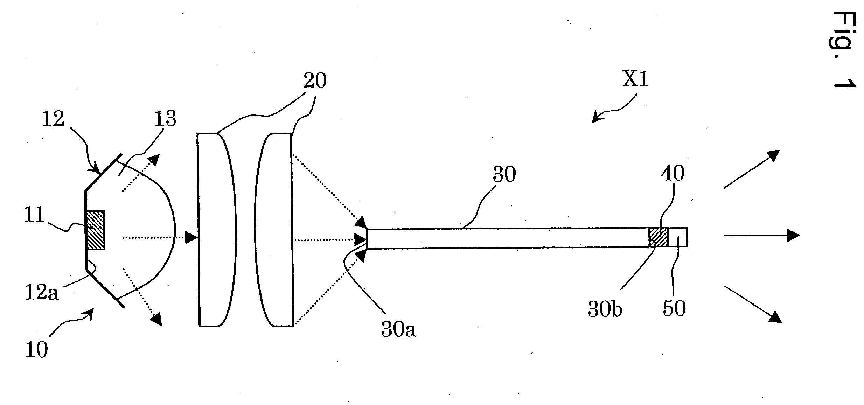

Fig. 1 schematically shows the light source apparatus X1 according to the first embodiment of the present invention. The light source apparatus X1 comprises a light source 10, a condenser lens 20, a light guide member 30, a wavelength selecting member 40 and a wavelength converting member 50.

The light source 10 comprises a semiconductor light emitting device 11, a reflecting member 12 and a sealing member 13, and emits light which excites the wavelength converting member 50 to be described later. The semiconductor light emitting device 11 emits light of a particular wavelength (short wavelength such as ultraviolet ray, for example, 390 nm or shorter). While the semiconductor light emitting device 11 may be an LED which is made of GaN-based compound semiconductor and emits in a range from blue light to ultraviolet ray, an edge emitting LED (EELED) or a super luminescent LED (SLED) is preferably used when emission with a particularly high brightness is required. The reflecting member 12 reflects light emitted by the semiconductor light emitting device 11, and is designed so as to reflect the light effectively toward the condenser lens 20 which will be described later. The reflecting member 12 of this embodiment is shaped substantially like a cup, and holds the semiconductor light emitting device 11 placed at the center of bottom surface thereof. The reflecting member 12 may be formed entirely from a metal or coated with aluminum or silver on the surface. The sealing member 13 is used to seal the semiconductor light emitting device 11 mounted on the reflecting member 12, and has translucency so as to transmit the light emitted by the semiconductor light emitting device 11. The sealing member 13 may also have the function of lens for concentrating the light emitted by the semiconductor light emitting device 11 onto the condenser lens 20.

The condenser lens 20, which may be either a single lens or a combination of a plurality of lenses (two lenses in this embodiment), concentrates the light emitted by the semiconductor light emitting device 11 of the light source 10 and introduces the light into the light guide member 30 at one end 30a thereof. The condenser lens 20 may be constituted from aspheric condenser lens or ball lens.

The light guide member 30 guides the light emitted by the semiconductor light emitting device 11 of the light source 10 to the wavelength converting member 50 which will be described later, and is constituted from an optical fiber or the like. The light guide member 30 may be formed from such a material as quartz glass, multi-component glass or plastics.

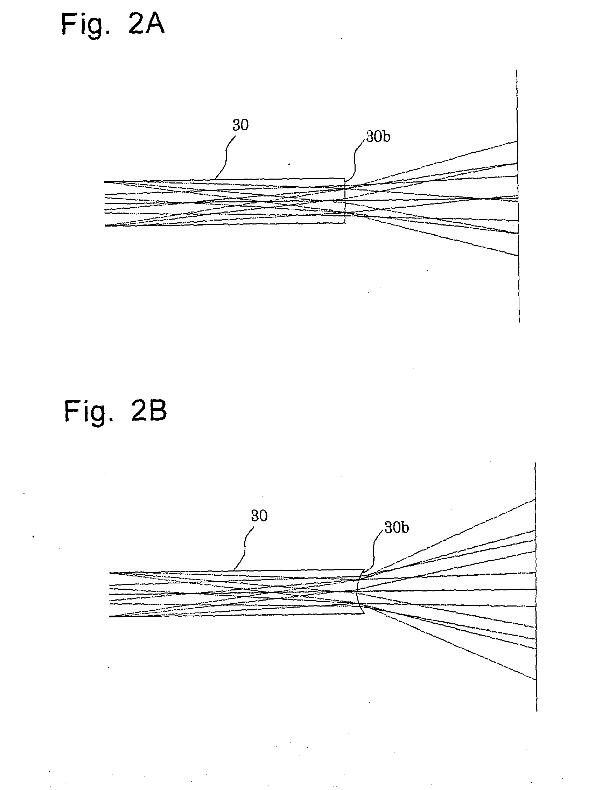

Fig. 2 shows the results of simulating the output beam resulting from the shape of the output end of the light guide member 30, Fig. 2A showing a case where the output end has flat configuration, and Fig. 2B showing a case where the output end has concave configuration (radius of curvature is 5 mm). The results of simulation shown in Fig. 2 indicate that the output beam in the case where the output end of the light guide member 30 has flat configuration spreads over an angle of 30°, and the output beam in the case where the output end of the light guide member 30 has concave configuration spreads over an angle of 50°. Therefore, in the light source apparatus X1, spread angle of the output beam can be increased (function of the illumination lens can be improved) by forming the output end of the light guide member 30 in concave shape. The spread angle of the output beam refers to the angle from the optical axis (direction where the radiation intensity is maximum) where the radiation intensity decreases to 50% of the maximum.

The wavelength selecting member 40 is disposed between the semiconductor light emitting device 11 of the light source 10 and the wavelength converting member 50 which will be described later (the other end 30b of the light guide member 30 in this embodiment), and reflects and transmits the incident light with a different reflectivity and a different transmittance depending on the wavelength of the incident light. The wavelength selecting member 40 may be a wavelength selecting filter (wavelength selecting mirror) or the like which includes a multi-layer dielectric film. The multi-layer dielectric film has such a constitution as dielectric film (λ/4 in thickness) having a relatively high refractive index and dielectric film (λ/4 in thickness) having a relatively low refractive index are placed alternately one on another, so as to show high reflectivity (for example, 90% or more) for light of a range of wavelengths including wavelength λ(for example, visible light) and high transmittance (for example, 90% or more) for light of wavelengths shorter than A (for example, ultraviolet ray). In case the wavelength selecting filter is used as the wavelength selecting member 40, the wavelength selecting member 40 may be disposed on the light guide member 30 by forming the multi-layer dielectric film on a glass-based substrate, cutting this into pieces of predetermined size by an ultrasound cutting machine or a dicing machine, and attaching the piece onto the other end 30b of the light guide member 30 with an adhesive or the like, or by forming the multi-layer dielectric film directly on the other end 30b of the light guide member 30 by vapor deposition or the like. In case the former method is employed, a matching oil (for example, silicone oil) may be interposed between the other end 30b of the light guide member 30 and the wavelength selecting member 40. While the size of the wavelength selecting member 40 may be determined in accordance to the spread angle of the beam of light emerging from the other end 30b of the light guide member 30, it is preferable to make the wavelength selecting member 40 larger than the other end 30b of the light guide member 30 so as to introduce more light via the wavelength selecting member 40 into the wavelength converting member 50, in case the beam spreads over a large angle.

An example of method for forming the multi-layer dielectric film directly on the other end 30b of the light guide member 30 will be described below. First, a fixture having a plurality of light guide members 30 held thereon is placed in a vapor deposition apparatus. Then a predetermined material to form the dielectric film is applied by vapor deposition onto the other end 30b of the light guide member 30 at a predetermined temperature (for example, from 100 to 150°C). In this way the multi-layer dielectric film can be formed on the other end 30b of the light guide member 30. In case an optical fiber held in a ferrule is used as the light guide member 30, the optical fiber and the ferrule may be polished together at the end faces thereof, with the polished face being coated with the multi-layer dielectric film by vapor deposition.

The wavelength converting member 50 receives the light emitted by the semiconductor light emitting device 11, and emits light having a wavelength longer than that of the original light and is formed, for example, by dispersing a phosphor, a pigment and the like in a resin (silicone resin) and curing the resin. In order to obtain white light from the wavelength converting member 50, such means may be employed as using a blue light emitting LED as the semiconductor light emitting device 11 and using a phosphor which emits yellow light (for example, YAG phosphor) as the wavelength converting member 50, or using an LED which emits violet light or ultraviolet ray as the semiconductor light emitting device 11 and using RGB phosphor (a combination of three phosphors whish emit red (R), green (G) and blue (B) light) as the wavelength converting member 50. In order to obtain white light having high color rendering property from the wavelength converting member 50, in particular, phosphors that constitute the RGB phosphor of predetermined proportions may be dispersed in the resin which is then cured. The output end of the wavelength converting member 50 may be formed in a shape similar to that of the output end of the light guide member 30.

The light source apparatus X1 of this embodiment has the wavelength converting member 40. Therefore, it is made possible to allow the light emitted by the semiconductor light emitting device 11 to pass through the wavelength selecting member 40 and reach the wavelength converting member 50 efficiently and effectively reflect the light directed toward the wavelength selecting member 40, among the light emerging from the wavelength converting member, by means of the wavelength selecting member 40, by setting a high transmittance (for example, 90% or more) for the wavelength of the light emitted by the semiconductor light emitting device 11 and setting a high reflectivity (for example, 90% or more) for the wavelength of the light emerging from the wavelength converting member 50. As a result, since the light emerging from the wavelength converting member 50 can be effectively extracted in the direction opposite to the wavelength selecting member 40 in the light source apparatus X1, the intensity of the optical output extracted in this direction can be increased. In case a member which includes a phosphor is used as the wavelength converting member 50, in particular, since the optical output from the phosphor has low directivity, the effect of increasing the output by the wavelength selecting member 40 can be achieved markedly.

The light source apparatus X1 is constituted such that light emitted by the semiconductor light emitting device 11 passes through the light guide member 30 and the wavelength selecting member 40 so as to be introduced into the wavelength converting member 50, and light emerging from the wavelength converting member 50 is extracted to the outside. As a result, the light emerging from the wavelength converting member 50 can be extracted to the outside without passing through the light guide member 30 in the light source apparatus X1. Thus the light source apparatus X1 is capable of improving the color rendering property of the light extracted therefrom, even when the light guide member 30 has transmission loss which is dependent on the wavelength.

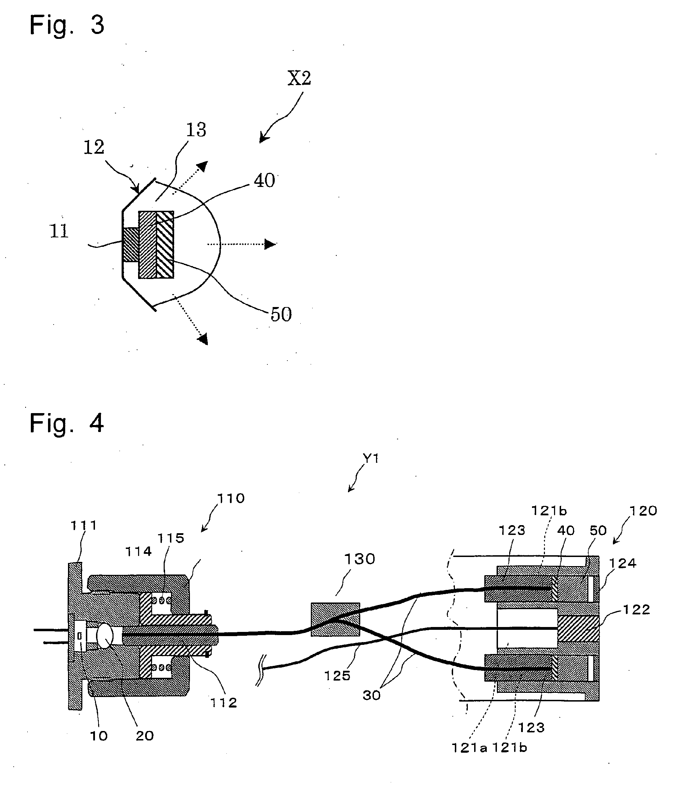

Fig. 3 schematically shows the constitution of the light source apparatus X2 according to the second embodiment of the present invention. The light source apparatus X2 is different from the light source apparatus X1 in that the wavelength selecting member 40 is attached directly onto the light emitting surface of the semiconductor light emitting device 11, excluding the condenser lens 20 and the light guide member 30, and that the wavelength selecting member 40 and the wavelength converting member 50 as well as the semiconductor light emitting device 11 are sealed with the sealing member 13. With other respect, constitution of the light source apparatus X2 is similar to that of the light source apparatus X1 described previously.

The light source apparatus X2 of this embodiment has the wavelength selecting member 40. Therefore, it is made possible to allow the light emitted by the semiconductor light emitting device 11 to pass through the wavelength selecting member 40 so as to reach the wavelength converting member 50 efficiently, and effectively reflect the light directed toward the wavelength selecting member 40, among the light emerging from the wavelength converting member 50, by means of the wavelength selecting member 40, by setting a high transmittance (for example, 90% or more) for the wavelength of the light emitted by the semiconductor light emitting device 11 and setting a high reflectivity (for example, 90% or more) for the wavelength of the light emerging from the wavelength converting member 50. As a result, since the light emerging from the wavelength converting member 50 can be effectively extracted in the direction opposite to the wavelength selecting member 40 in the light source apparatus X2, the intensity of the optical output extracted in this direction can be increased. In case a member which includes a phosphor is used as the wavelength converting member 50, in particular, since the optical output from the phosphor has low directivity, the effect of increasing the output power by the wavelength selecting member 40 can be achieved more markedly.

Also in the light source apparatus X2, the semiconductor light emitting device 11 and the wavelength converting member 50 are connected to each other via the wavelength selecting member 40. In other words, the light source apparatus X2 does not have a member, such as the light guide member 30 having transmission loss that is dependent on the wavelength, which is interposed between the semiconductor light emitting device 11 and the wavelength converting member 50. Thus the light source apparatus X2 is capable of improving the color rendering property of the light extracted therefrom.

An example of method for manufacturing the light source apparatus X2 will now be described.

First, the wavelength selecting member 40 is disposed on the light emitting surface of the semiconductor light emitting device 11, which may be carried out by forming the multi-layer dielectric film on a glass-based substrate, cutting this into pieces of a predetermined size, and attaching the piece onto the light emerging face of the semiconductor light emitting device 11, or by forming the multi-layer dielectric film directly on the light emitting surface of the semiconductor light emitting device 11 by vapor deposition or the like.

Then the semiconductor light emitting device 11 having the wavelength selecting member 40 attached thereto is placed at a predetermined position (at the bottom of cup in this embodiment) of the reflecting member 12.

Then the wavelength converting member 50 is formed on the wavelength selecting member 40. Specifically, a predetermined amount of material (for example, silicone resin including a phosphor) is dipped on the wavelength selecting member 40, and is left to stand at the room temperature or heated at a predetermined temperature (for example, 60°C) so as to cure the material. Also a part of the material may be caused to flow onto the side face of the wavelength selecting member by dipping.

Then the semiconductor light emitting device 11, the wavelength selecting member 40 and the wavelength converting member 50 are sealed over the entire surfaces hereof with the sealing member 13. Thus the light source apparatus X2 shown in Fig. 3 is made.

Fig. 4 is a partial sectional view schematically showing the constitution of the endoscope Y1 according to the third embodiment of the present invention. The endoscope Y1 comprises the light guide member 30, a light source connector 110, an image pickup device mounting section 120 and branching means 130, and is inserted into, for example, a body cavity so as to investigate the situation of a body organ. Members of the endoscope Y1 which are identical or similar to those of the light source apparatus X1 will be identified by the same reference numerals.

The light source connector 110 has the light source 10, the condenser lens 20, a holder 111, a ferrule 112, a holder 113 and linkage means 114, where the light source 10, the condenser lens 20 and one end of the light guide member 30 are disposed in predetermined positional relationship. The holder 111 is provided to support the light source 10 and the condenser lens 20, so that light emitted by the light source 10 is concentrated by the condenser lens 20 and is introduced into the light guide member 30 at one end thereof. One end of the light guide member 30 is inserted into a through hole of the ferrule 112, and is secured by means of, for example, an adhesive which is not shown. The holder 113 supports the ferrule 112. The linkage means 114 is provided to support the holder 113 in a slidable state via an elastic member 115, and links the holder 111 and the holder 113 with each other. Linkage of the holder 111 and the holder 113 by the linkage means 114 can be canceled as required.

The image pickup device mounting section 120 comprises the wavelength selecting member 40, the wavelength converting member 50, the holder 121, an image pickup device 122, a ferrule 123 and a protective member 124, where the other end of the light guide member 30, the wavelength selecting member 40 and the wavelength converting member 50 are disposed in predetermined positional relationship. The holder 121 has substantially cylindrical shape and has a through hole 121a positioned at the center thereof, and through holes 121b (two holes in this embodiment) positioned so as to interpose the through hole 121a. The image pickup device 121 is a semiconductor device which has the function of photo-electric conversion, and is secured in the through hole 121a by means of an adhesive or a precision screw which is not shown. The image pickup device 121 is positioned substantially at the center of the image pickup device mounting section 120, and is controlled by control means (not shown) which is connected via a control line 125. The image pickup device 121 may be an image sensor based on CCD (charge coupled device) or on CMOS (complementary metal oxide semiconductor). Inserted into the through hole of the ferrule 122 is the other end of the light guide member 30 and secured by means of an adhesive, and the ferrule 122 is secured in the through hole 121b by means of an adhesive or a precision screw which is not shown. Provided on the distal end of the ferrule 122, wherein the other end of the light guide member 30 is inserted and secured, are the wavelength selecting member 40 and the wavelength converting member 50 formed thereon one on another. The protective member 124, which protects the wavelength selecting member 40 and the wavelength converting member 50 provided on the distal end of the ferrule 122 from the environmental influences, is attached at the end of the through hole 121b. The protective member 124 is preferably constituted so that the spread angle of the light beam which emerges through the protective member 124 has a proper extent for the endoscope Y1 (for example, 120° or more), and is formed in, for example, a concave surface at the end thereof where the light emerges, for a similar reason described in relation to the simulation of the light guide member 30 described previously. The protective member 124 may be constituted from a cover glass, for example.

An example of forming the wavelength selecting member 40 and the wavelength converting member 50 provided on the end of the ferrule 122 will now be described below. The wavelength selecting member 40 attached to the distal end of the ferrule 122, on which the other end of the light guide member 30 is inserted and secured, is inserted into the through hole 121b of the holder 121, and is secured therein by means of an adhesive, a precision screw or the like. Then with the holder 121 placed in such a posture as the wavelength selecting member 40 faces upward, a predetermined amount of material for forming the wavelength converting member 50 (for example, silicone resin including a phosphor) is dipped on the wavelength selecting member 40, and is left to stand at the room temperature or heated at a predetermined temperature (for example, 60°C) so as to cure the material. The wavelength converting member 50 is thus formed. While thickness of the wavelength converting member 50 is set to about 1 mm, the present invention is not limited to this and various thicknesses may be selected depending on the kind of phosphor and the amount thereof dispersed in the resin.

The branching means 130 branches the light guide member 30 amid the length thereof, in order to introduce the light emerging from the light source connector 110 into two wavelength converting members 50. Proportions of the beams of light split by the branching means 130 are preferably near even (50% in this embodiment), in order to make the brightness and output power of light emerging from the wavelength converting member 50 to the outside uniform. The branching means 130 may be constituted from, for example, a photocoupler.

The endoscope Y1 of this embodiment incorporates a constitution similar to that of the light source apparatus X1, and therefore achieves an effect similar to that of the light source apparatus X1. Also because the endoscope Y1 has the image pickup device 121, it provides an image of the area, which is illuminated by the light emerging from the wavelength converting member 50, in the form of electrical signals converted by the image pickup device 121.

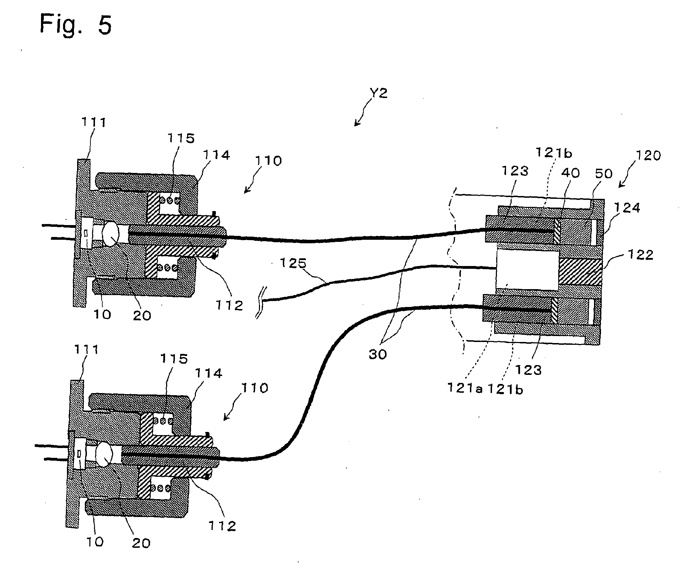

Fig. 5 is a partial sectional view schematically showing the constitution of the endoscope Y2 according to the fourth embodiment of the present invention. The endoscope Y2 is different from the endoscope Y1 in that the light guide member 30 and the light source connector 110 are each provided in two lines, instead of using the branching means 130. With other respects, the endoscope Y2 has constitution similar to that of the endoscope Y1. Three or more units of each of the light guide member 30 and the light source connector 110 may also be provided.

The Y2 of this embodiment allows it to increase the optical output from the wavelength converting member 50 to about two times (depending on the number of units descried above) that of the endoscope Y1. Also because at least of the light sources can be kept as backup in the endoscope Y2, light output can be maintained for the purpose of illumination even when the main light source shuts down during use.

Specific embodiments of the present invention have been described above, although the present invention is not limited to these embodiments, and various modifications may be made without deviating from the scope of the invention.

The light source 10 may be of such a type as the light diverges less (for example, a light source that emits laser beam) in the light source apparatuses X1, X2 and the endoscopes Y1, Y2. Such a constitution makes it possible to introduce light of relatively high intensity into the wavelength converting member 50 via the wavelength selecting member 40 without increasing the size of the wavelength selecting member 40.

In the light source apparatus X1 and the endoscopes Y1, Y2, an optical fiber having lens function (such as grated index fiber) may be provided between the other end 30b of the light guide member 30 and the wavelength selecting member 40. With such a constitution, since the light sent from the optical fiber toward the wavelength selecting member 40 can be suppressed from spreading by the lens function of the optical fiber, it is made possible to introduce light of relatively high intensity into the wavelength converting member 50 via the wavelength selecting member 40 without increasing the size of the wavelength selecting member 40.

The endoscopes Y1, Y2 may employ an image guide fiber designed for use in a fiber scope, instead of the image pickup device 121 and the control line 125. Such a constitution is preferable for making the image pickup device mounting section 120 of the endoscopes Y1, Y2 smaller in size. The endoscopes Y1, Y2 may also be used as illumination apparatus without any of the image pickup device 121 and a control line 125 and the image guide fiber designed for fiber scope provided therein.

The light source apparatuses X1, X2 have high output power and high color rendering property, and therefore may be used as the light source apparatus for image projector, automobile headlamp, and various biomedical equipments. While an image projector requires a point light source having high output power, the light emerging end face of the light source apparatus X1 is preferably used as a point light source.

### EXAMPLES

### Fabrication of light source apparatus

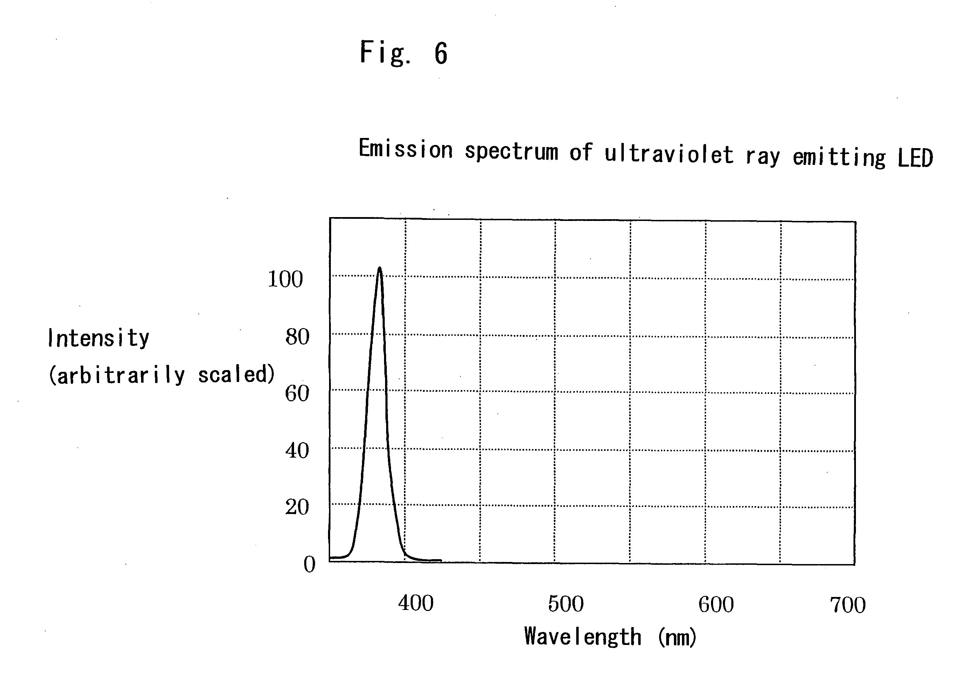

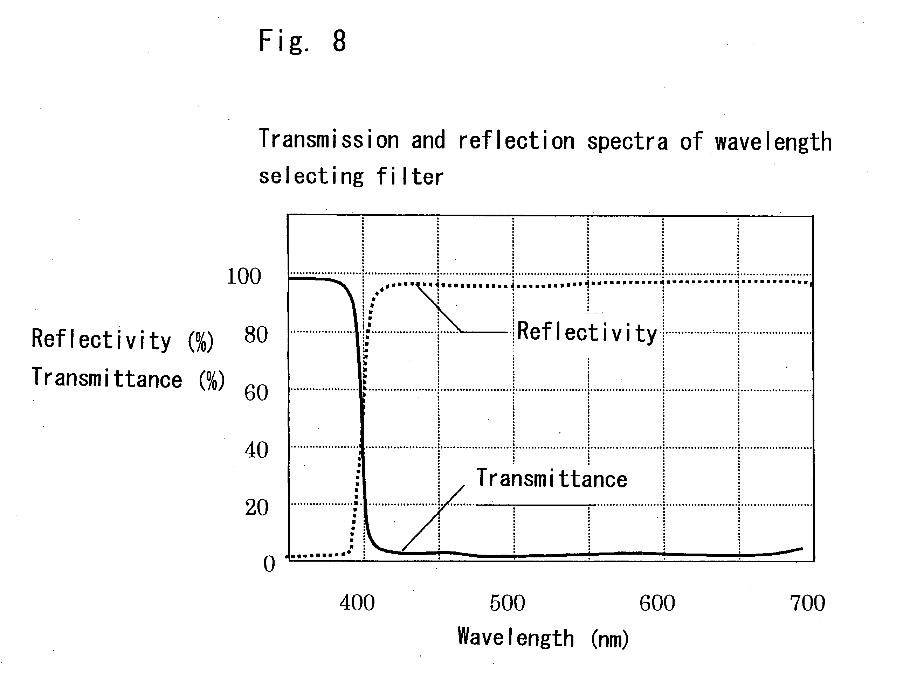

A light source apparatus having a constitution similar to that of the light source apparatus X1 shown in Fig. 1 was made, using an ultraviolet ray emitting LED as the semiconductor light emitting device 11. Fig. 6 is the emission spectrum of the ultraviolet ray emitting LED. As clearly shown in Fig. 6, wavelengths of light emitted by the ultraviolet ray emitting LED were concentrated around 380 nm. The reflecting member 12 having aluminum coating was used. A resin was used as the sealing member 13. The sealing member 13 was formed in such a constitution that has lens function to converge the beam of light emitted by the ultraviolet ray emitting LED with a smaller angle of spread. An aspheric lens was used as the condenser lens 20. An acrylic optical fiber (2 mm in diameter and 3 m in length) was used as the light guide member 30. Fig. 7 shows the spectrum of transmission loss of the acrylic optical fiber. A wavelength selecting filter constituted from a multi-layer dielectric film was used as the wavelength selecting member 40. Fig. 8 is a diagram showing the transmission and reflection spectra of the wavelength selector mirror. The wavelength selecting filter was designed to have a transmittance of 90% (a level deemed sufficient) or higher for the wavelength (about 380 nm) of light emitted by the ultraviolet ray emitting LED and a reflectivity of 90% (a level deemed sufficient) or higher for the wavelength (about 410 to 700 nm) of white light. For the wavelength converting member, a member including phosphor made by mixing RGB phosphor in silicone resin was used. The RGB phosphor included in the phosphor-including member was constituted from La₂O₂S doped with Euro (R component), SrAl₂O₂ doped with Euro (G component) and BaMgAl₁₀O₁₇ doped with Euro (B component).

### Evaluation

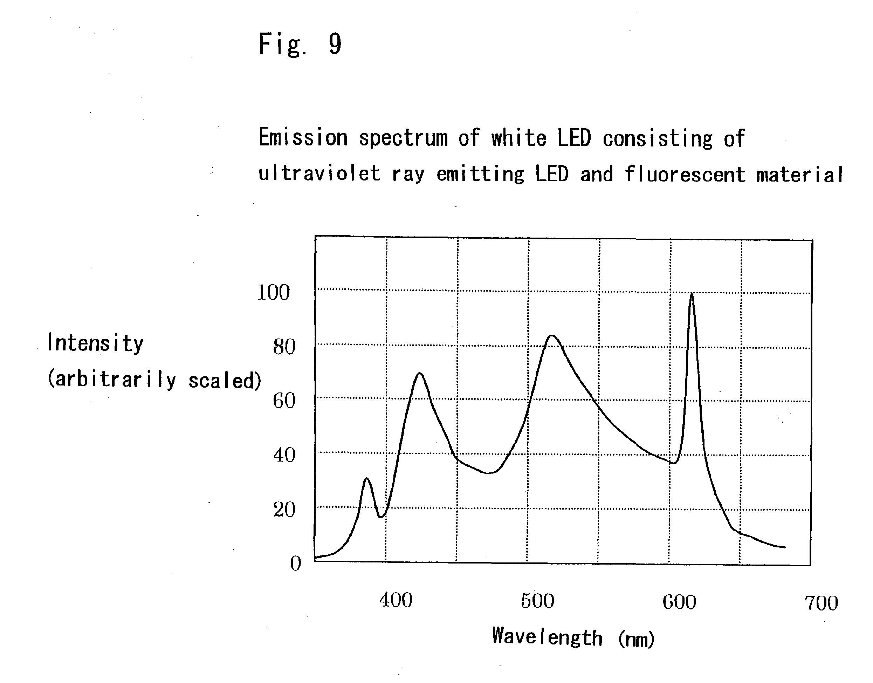

Fig. 9 shows emission spectrum of the light source apparatus of this Example fabricated as described above. In the light source apparatus of the prior art, since white light having such a spectrum as shown in Fig. 5 is output to the outside through an optical fiber having such a transmission spectrum as shown in Fig. 7, the optical output has such a spectrum that the intensity is relatively low at wavelengths around 630 nm, thus it is difficult to maintain high color rendering property. The light source apparatus of this Example, in contrast, is not substantially affected by the wavelength dependency of the transmission loss of the optical fiber made of acrylic resin, thus making it possible to maintain high color rendering property. Also with the light source apparatus of this Example, it was verified that output power higher than that of the light source apparatus of the prior art can be obtained by using the wavelength selecting filter. From these results, it can be concluded that the light source apparatus of this Example has higher output power and better color rendering property than the light source apparatus of the prior art.

## Claims

1. A light source apparatus comprising:
a semiconductor light emitting device which emits light;
a wavelength converting member which receives the light emitted by the semiconductor light emitting device and emits light of a wavelength longer than that of the original light; and
a wavelength selecting member which is disposed between the semiconductor light emitting device and the wavelength converting member and reflects and transmits the incident light with different reflectivity and different transmittance depending on the wavelength of the incident light.

2. The light source apparatus according to claim 1,
wherein the wavelength selecting member has a transmittance not less than 90% for the wavelength of the light emitted by the semiconductor light emitting device and a reflectivity not less than 90% for the wavelength of the light emerging from the wavelength converting member.

3. The light source apparatus according to claim 1,
wherein the wavelength selecting member has a transmittance not less than 90% for the wavelength of ultraviolet ray and a reflectivity not less than 90% for the wavelength of visible ray.

4. The light source apparatus according to claim 1,
wherein the semiconductor light emitting device mainly emits light of a wavelength not more than 390 nm and the wavelength converting member mainly emits light of a wavelength in a range from 410 nm to 700 nm.

5. The light source apparatus according to claim 1,
wherein the wavelength selecting member has a multi-layer dielectric film.

6. The light source apparatus according to claim 1,
wherein the wavelength converting member has a phosphor.

7. A light source apparatus comprising:
a semiconductor light emitting device which emits light;
a wavelength converting member which receives the light emitted by the semiconductor light emitting device and emits light of wavelength longer than the original light; and
a light guide member which guides the light emitted by the semiconductor light emitting device to the wavelength converting member.

8. The light source apparatus according to claim 7, further comprising a wavelength selecting member which is disposed between the semiconductor light emitting device and the wavelength converting member and reflects and transmits the incident light with different reflectivity and different transmittance depending on the wavelength of the incident light.

9. The light source apparatus according to claim 8,
wherein the wavelength selecting member has a transmittance not less than 90% for the wavelength of the light emitted by the semiconductor light emitting device and a reflectivity not less than 90% for the wavelength of the light emerging from the wavelength converting member.

10. The light source apparatus according to claim 8,
wherein the wavelength selecting member has a transmittance not less than 90% for the wavelength of ultraviolet ray and a reflectivity not less than 90% for the wavelength of visible ray.

11. The light source apparatus according to claim 8,
wherein the semiconductor light emitting device mainly emits light of a wavelength not more than 390 nm and the wavelength converting member mainly emits light of a wavelength in a range from 410 nm to 700 nm.

12. The light source apparatus according to claim 8,
wherein the wavelength selecting member has a multi-layer dielectric film.

13. The light source apparatus according to claim 7,
wherein the wavelength converting member has a phosphor.

14. The light source apparatus according to claim 7,
wherein the light guide member is a optical fiber.

15. The light source apparatus according to claim 7,
wherein the optical fiber is made of plastics, and
wherein the semiconductor light emitting device mainly emits light of a wavelength in a range from 400 nm to 600 nm and the wavelength converting member emits visible light.

16. The light source apparatus according to claim 7,
wherein an output end of the light guide member has a concave configuration portion.

17. The light source apparatus according to claim 7,
wherein an output end of the wavelength converting member has a concave configuration portion.

18. An endoscope comprising:
the light source apparatus as in one of claims 1 - 18; and
an image pickup device which captures the image of an area illuminated by the light emitted by the light source apparatus.
